# EUROPEAN PATENT APPLICATION

(11) **EP 3 017 777 A1**
(43) Date of publication of application: **11.05.2016**
(21) Application number: 15192557.5
(22) Date of filing: 02.11.2015
(51) Int. Cl.: A61B 17/34

(54) **NEEDLE DEVICE AND NEEDLE SYSTEM HAVING THE SAME**

(30) Priority: 07.11.2014 TW 103138811
(71) Applicant: EDA Medical Devices Technology Inc., Taichung City 428 (TW)
(72) Inventor: CHIANG, Tai-An, 428 Taichung City (TW); UEN, Tzeng-Ming, 428 Taichung City (TW)
(74) Representative: Schweiger, Johannes

(57) **Abstract**

A needle device (1) includes a shell (2), a needle unit (3) and an actuator (5) . The needle unit (3) includes an outer needle guide (32) and a needle member (31). The outer needle guide (32) has a piercing end (321) for penetrating into a biological body part. The needle member (31) is disposed in the shell (2) and the outer needle guide (32), has a needle tip (312) protruding from a front end (21) of the shell (2) and is movable relative to the shell (2) and the outer needle guide (32). The actuator (5) is responsive to a control signal to drive the needle member (31) and move the needle tip (312) between a protruding position, where the needle tip (312) prctrudes out of the piercing end (321) of the outer needle guide (32), and a retracing position, where the needle tip (312) is retracted back into the outer needle guide (32).

## Description

The disclosure relates to a needle device and a needle system having the same, and more particularly to a needle device for identifying tissue type of a tissue layer and a needle system having the same.

Advancements in medical technology have increased success rates of medical surgeries in general, but there are still types of medical surgeries which solely rely on proficiencies of human operators. Taking one of the commonly-practiced minimally invasive procedures, laparoscopic surgery, as an example, the peritoneal cavity of a patient is usually insufflated with carbon dioxide gas using a trocar needle to create a working space for the surgical procedure. However, since the trocar needle is usually inserted blindly, the trocar needle may puncture an internal organ by accident and undesirably lead to a fully open surgical procedure.

Another example of the medical surgeries is epidural anesthetic, which is commonly employed, especially in childbirth for both vaginal and caesarean deliveries, to produce loss of sensation below the waist. Since the epidural space is a narrow space between the dura mater and the ligamentum flavum which ranges from 1 to 6 mm in width depending on the size and/or age of a patient, accurate injection of anesthetics into the epidural space requires the operator to be both technically proficient and experienced. Great care must be taken to avoid puncturing the dura, which may lead to complications such as post-dural puncture headache (PDPH) and may even damage the spinal cord. Therefore, a tool, which can provide a quantifiable judging standard and which can aid the operator in precise position determination, is needed.

An object of the disclosure is to provide a needle device that can assist in identification of a tissue type of a tissue layer.

According to one aspect of the disclosure, the needle device includes a shell, a needle unit and an electrically operable actuator.

The shell has front and rear ends opposite to each other along a needle axis.

The needle unit includes an outer needle guide and a needle member. The outer needle guide is mounted to the front end of the shell, extends along the needle axis, spatially communicates with the shell, and has a piercing end configured to penetrate into a biological body part. The needle member is disposed in the shell and the outer needle guide, has a needle tip protruding from the front end of the shell, and is movable relative to the shell and the outer needle guide.

The actuator is responsive to a control signal to drive the needle member and move the needle tip between a protruding position, where the needle tip protrudes out of the piercing end of the outer needle guide, and a retracting position, where the needle tip is retracted back into the outer needle guide.

Another object of the disclosure is to provide a needle system for monitoring penetration into a biological body part, identifying tissue type of a tissue layer, and confirming arrival at a target tissue layer.

According to another aspect of the disclosure, the needle system includes a needle device for penetrating into a biological body part, and a control module.

The needle device includes a needle member that extends and is movable along a needle axis and that has a needle tip, and an electrically operable actuator that is responsive to a control signal to drive the needle member to move along the needle axis against a resistance force from a tissue layer being penetrated by the needle device.

The control module includes a displacement detecting unit that is operable to detect displacement of the needle member along the needle axis, and a control unit that is electrically coupled to the actuator and the displacement detecting unit, that is operable to output the control signal to the actuator, and that is operable to identify the tissue type of the tissue layer based on the control signal and the displacement of the needle member detected by the displacement detecting unit.

Other features and advantages of the disclosure will become apparent in the following detailed description of the embodiments with reference to the accompanying drawings, of which:
FIG. 1 is an exploded perspective view illustrating a first embodiment of a needle device included in a needle system according to the disclosure;
FIG. 2 is an assembled perspective view of the needle device of the first embodiment;
FIG. 3 is a schematic block diagram illustrating a control module of the first embodiment;
FIG. 4 is a fragmentary enlarged perspective view of the needle device of the first embodiment, with an outer needle guide and a mounting seat being omitted;
FIG. 5 is a fragmentary sectional view of the first embodiment;
FIG. 6 is a view similar to FIG. 5 but sectioned from another angle, illustrating a needle member at a protruding position;
FIG. 7 is a view similar to FIG. 6, illustrating the needle member at a retracting position;
FIG. 8 is a view similar to FIG. 6, illustrating that the needle member of the first embodiment has penetrated into a biological body part;
FIG. 9 is a fragmentary sectional view illustrating a second embodiment of the needle system according to the disclosure; and
FIG. 10 is a view similar to FIG. 9, illustrating that a needle member of the second embodiment has penetrated into a biological body part.

Before the disclosure is described in greater detail, it should be noted that like elements are denoted by the same reference numerals throughout the disclosure.

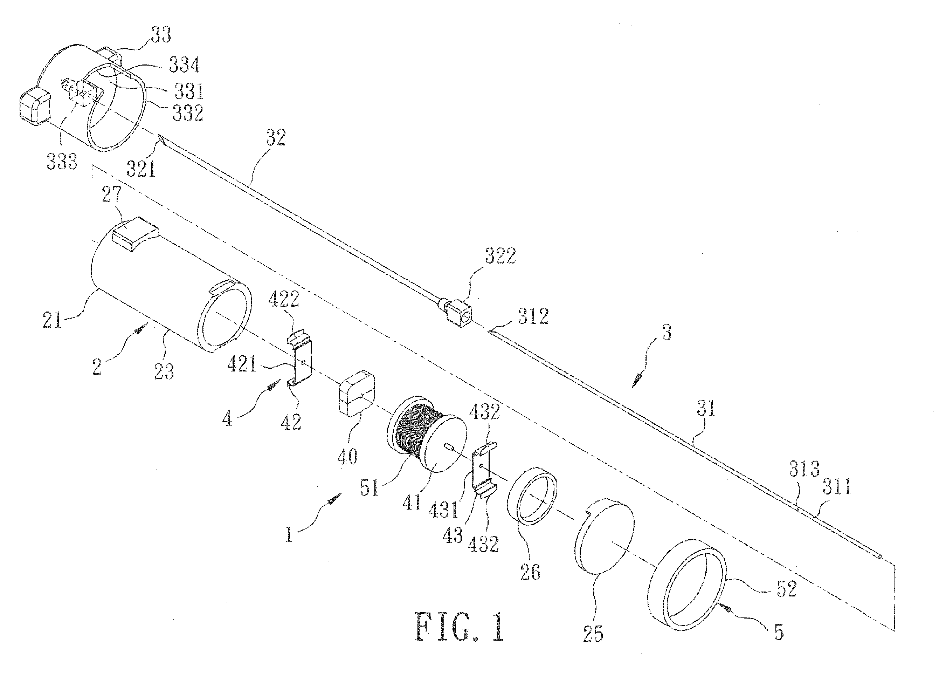

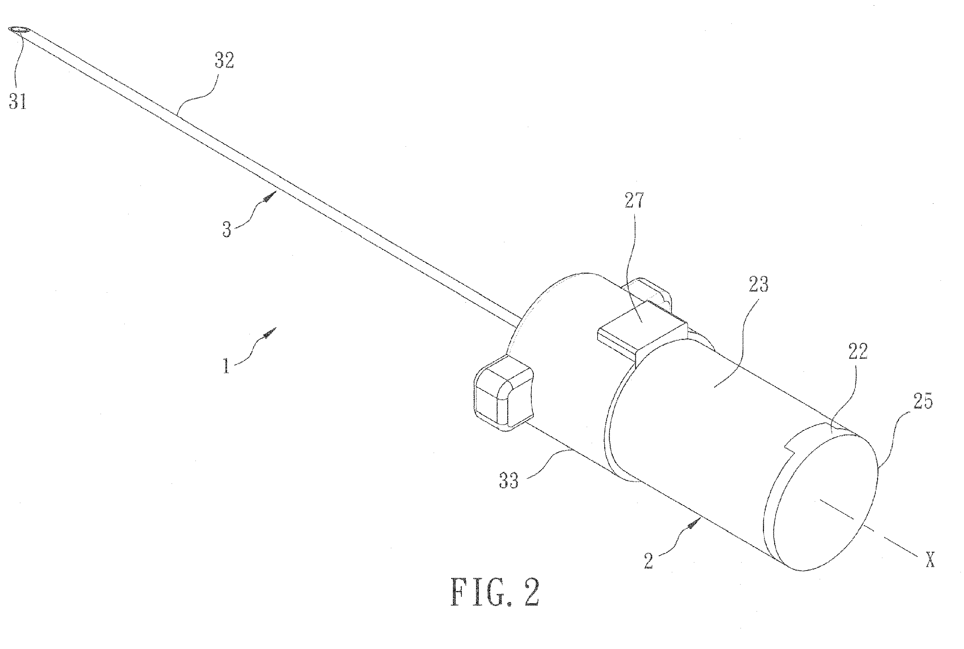

Referring to FIGS. 1 to 8, a first embodiment of a needle system according to this disclosure is capable of identifying tissue type of a tissue layer and is shown to include a needle device 1 for penetrating into a biological body part, and a control module 10.

The needle device 1 includes a shell 2, a needle unit 3, an electrically operable actuator 5 and a biasing unit 4.

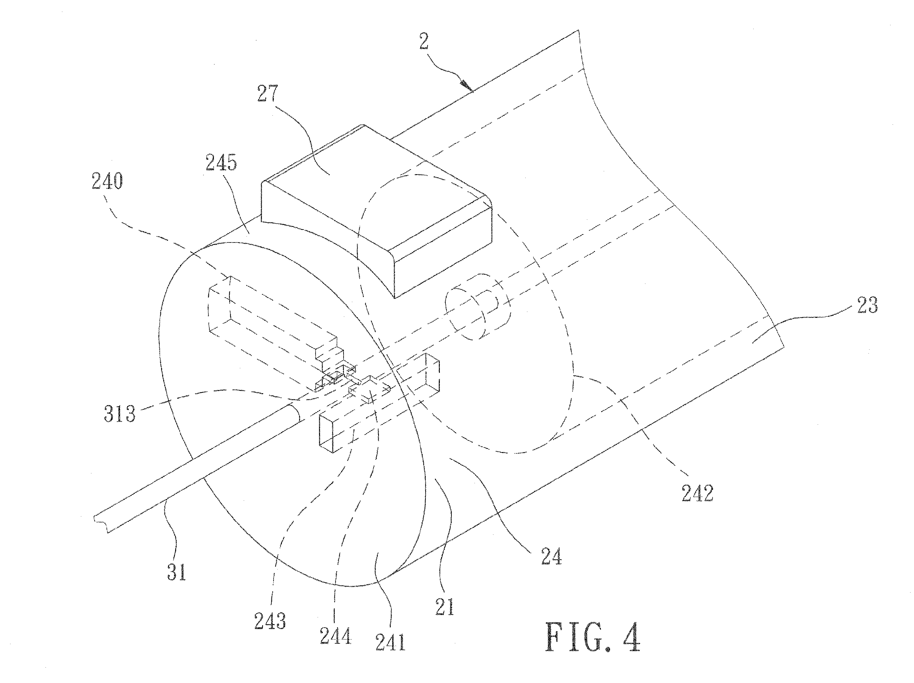

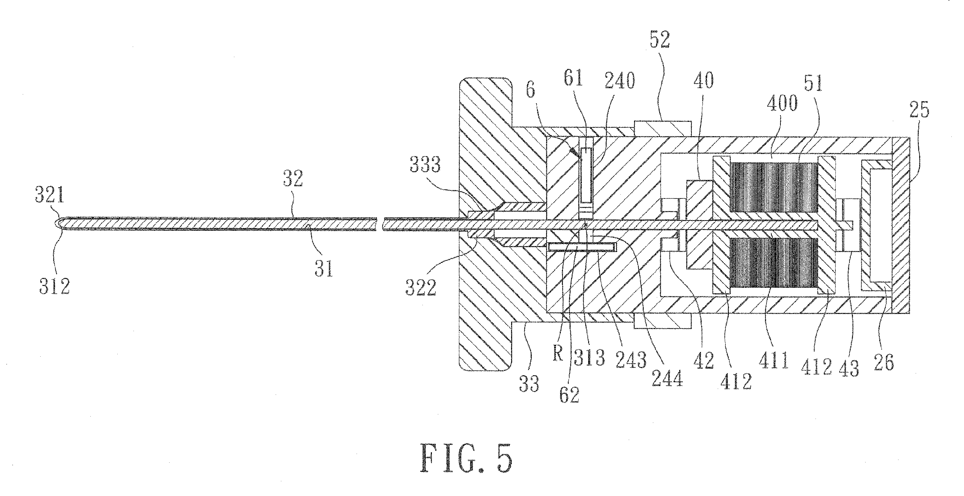

The shell 2 has front and rear ends 21, 22 opposite to each other along a needle axis (X) and includes a tubular body 23 surrounding the needle axis (X), a front wall 24 disposed at a front end of the tubular body 23, a cover 25 removably covering a rear end of the tubular body 23, and a fixed reference (R) located in the front wall 24 (see FIG. 5). The front wall 24 has a surrounding surface 245 (see FIG. 4) that surrounds the needle axis (X), front and rear end surfaces 241, 242 that are opposite to each other along the needle axis (X), and a first engaging portion 27 that is configured as a protrusion and that protrudes outwardly from the surrounding surface 245. In this embodiment, a front end of the front wall 24 constitutes the front end 21 of the shell 2, and a rear end of the cover 25 constitutes the rear end 22 of the shell 2.

The needle unit 3 includes an outer needle guide 32, a needle member 31 and a mounting seat 33. The outer needle guide 32 is mounted to the front end 21 of the shell 2, extends along the needle axis (X) to surround the needle member 31, and spatially communicates with the shell 2. In this embodiment, the outer needle guide 32 has a piercing end 321 configured to penetrate into a biological body part and a mounting portion 322 opposite to the piercing end 321 along the needle axis (X). To be more specific, the mounting portion 322 of the outer needle guide 32 is mounted to the mounting seat 33. The needle member 31 extends along the needle axis (X) and is disposed in the shell 2 and the outer needle guide 32. In other words, the shell 2 and the outer needle guide 32 cooperatively receive the needle member 31. The needle member 31 has a needle tip 312 protruding from the front end 21 of the shell 2, and is movable along the needle axis (X) relative to the shell 2 and the outer needle guide 32. The needle member 31 further has a receiving portion 311 that is disposed in the shell 2 and that is provided with a positioning reference 313. The mounting seat 33 includes a base wall 331 that is formed with a groove 333 for accommodating the mounting portion 322 of the outer needle guide 32 therein, and a surrounding wall 332 that is formed with a second engaging portion 334 configured as a notch and engaging the first engaging portion 27 of the front wall 24 of the shell 2 so that the mounting seat 33 is stably mounted onto the front end 21 of the shell 2, thereby allowing the outer needle guide 32 to be fixedly mounted to the shell 2.

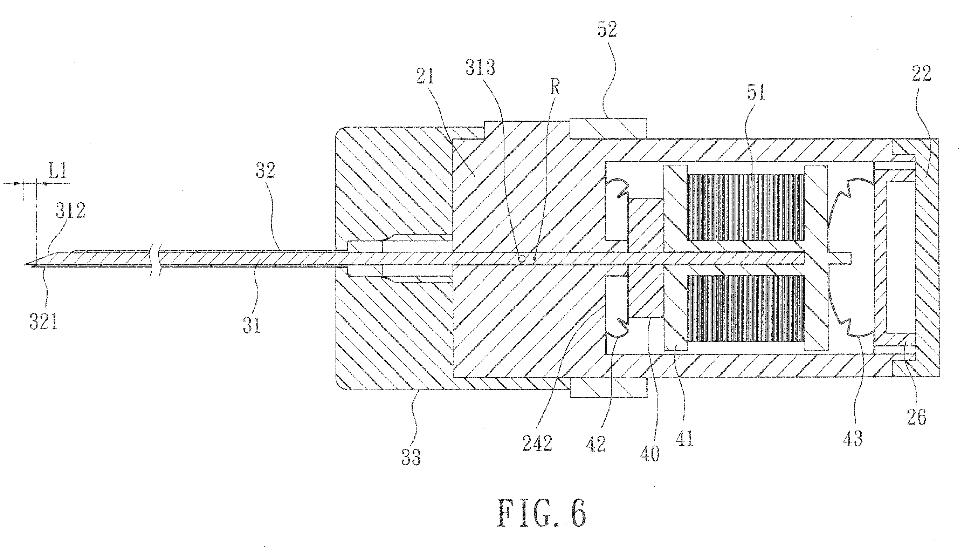

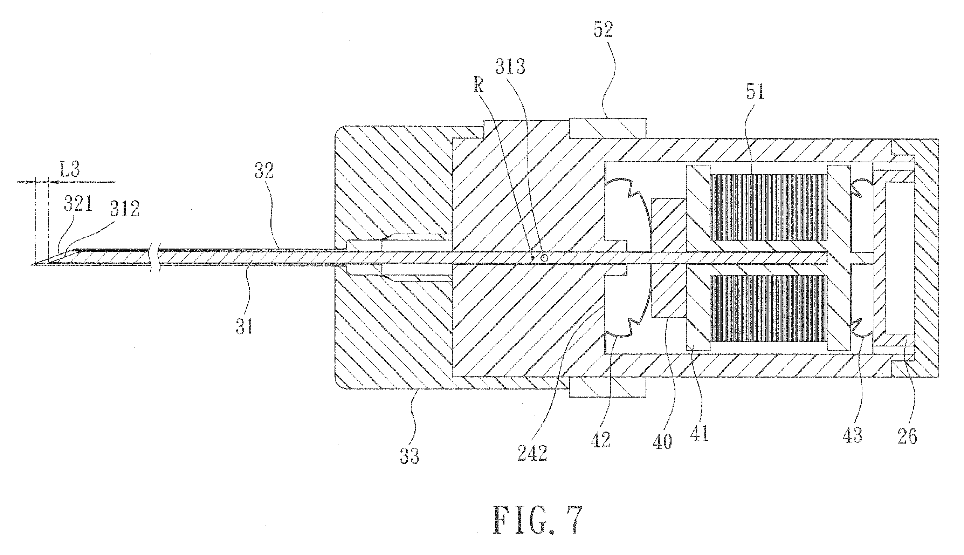

The actuator 5 is responsive to a control signal to drive the needle member 31 to move along the needle axis (X) against a resistance force from a tissue layer being penetrated by the needle device 1. The actuator 5 is operable to drive the needle member 31 and move the needle tip 312 relative to the outer needle guide 32 between a protruding position, where the needle tip 312 protrudes out of the piercing end 321 of the outer needle guide 32 (e.g., FIG. 6), and a retracting position, where the needle tip 312 is retracted back into the outer needle guide 32 (e.g., FIG. 7) . In this embodiment, the actuator 5 includes a first magnetic element 51 that is connected to the needle member 31, and a second magnetic element 52 that is mounted to the shell 2 and that cooperates with the first magnetic element 51 for driving movement of the needle member 31 along the needle axis (X). In this embodiment, the first magnetic element 51 is configured as a coil that is disposed in the shell 2 and that is responsive to polarity and amplitude of the control signal, and the second magnetic element 52 is configured as a magnet that surrounds the tubular body 23 of the shell 2 and that provides a magnetic field. Since the technique generating a driving force to drive movement of the needle member 31 by cooperation between the first and second magnetic elements 51, 52 should be readily appreciated by those skilled in the art, further detail will not be provided herein for the sake of brevity. As long as one of the first magnetic element 51 and the second magnetic element 52 is configured as a coil that is responsive to the control signal, the other one of the first and second magnetic elements 51, 52 may be configured as a coil, a magnet, etc. with no particular preferences. When both magnetic elements 51, 52 are coils, they both receive the control signal.

Referring to FIGS. 1, 5 and 6, the biasing unit 4 is disposed in the shell 2, is connected to the needle member 31, and biases the needle member 31 against the driving force provided by the actuator 5. The biasing unit 4 includes a connecting member 41, a first resilient member 42 and a second resilient member 43. The connecting member 41 is connected to the needle member 31 and is slidable along the needle axis (X) relative to the shell 2. The first and second resilient members 42, 43 are respectively disposed on two opposite sides of the connecting member 41 along the needle axis (X). The first resilient member 42 has two opposite ends respectively and persistently abutting against the connecting member 41 and the front end 21 of the shell 2. The second resilient member 43 has two opposite ends respectively and persistently abutting against the connecting member 41 and the rear end 22 of the shell 2.

To be more specific, the first resilient member 42 has a main portion 421 and two leg portions 422 respectively extending from two opposite sides of the main portion 421, and the second resilient member 43 has a main portion 431 and two leg portions 432 respectively extending from two opposite sides of the main portion 431. The needle device 1 is provided with a first abutment piece 40 disposed between the first resilient member 42 and the connecting member 41, and a second abutment piece 26 disposed in the shell 2 and adjacent to the cover 25. The main portion 421 and the leg portions 422 of the first resilient member 42 respectively and persistently abut against the first abutment piece 40 and the rear end surface 242 of the front wall 24 of the shell 2. The main portion 431 and the leg portions 432 of the second resilient member 43 respectively and persistently abut against the connecting member 41 and the second abutment piece 26 (see FIG. 6). The first and second abutment pieces 40, 26 are configured to restrict deformation of the first and second resilient members 42, 43 within a predetermined allowable extent so as to prevent a sudden uncontrollable movement of the needle member 31 due to over-stretching of one of the first and second resilient members 42, 43. Alternatively, in other variations of this embodiment, the size of the shell 2 may be adjusted, and the first and second abutment pieces 40, 26 may be omitted.

As best shown in FIG. 5, the connecting member 41 includes a cylinder body 411 that extends along the needle axis (X) and that surrounds the needle member 31, and two side plates 412 that are respectively connected to two opposite ends of the cylinder body 411 and that cooperate with the cylinder body 411 to confine an accommodating space 400 for accommodating the first magnetic element 51 of the actuator 5.

Referring back to FIGS. 1, 3 and 5, the control module 10 includes a displacement detecting unit 6, a control unit 8 and a monitoring unit 9.

The displacement detecting unit 6 is operable to detect displacement of the needle member 31 along the needle axis (X) based on displacement of the positioning reference 313 of the needle member 31 relative to the fixed reference (R) of the shell 2. In this embodiment, the displacement detecting unit 6 includes a light emitting member 61 disposed adjacent to the positioning reference 313, and a light detecting member 62 disposed corresponding in position to the light emitting member 61 and the positioning reference 313 for detecting displacement of the positioning reference 313 relative to the fixed reference (R) . In this embodiment, the light detecting member 62 is a charge-coupled device.

As shown in FIGS. 4 and 5, the positioning reference 313 is configured as a radially-extending hole in this embodiment. The front wall 24 of the shell 2 has the needle member 31 extending therethrough. The surrounding surface 245 of the front wall 24 is formed with a first slot 240 which extends radially inward and which accommodates the light emitting member 61 therein. The front end surface 241 is formed with a second slot 243 which is opposite to the first slot 240 in a direction transverse to the needle axis (X), which extends away from the outer needle guide 32 and which accommodates the light detecting member 62, and a connecting slot 244 which is formed between the positioning reference 313 of the needle member 31 and the second slot 243. The first slot 240 spatially communicates with the second slot 243 via the positioning reference 313 and the connecting slot 244. Alternatively, in other variations of this embodiment, the positioning reference 313 may be configured as a mark, and the light emitting member 61 and the light detecting member 62 may be disposed in one of the first slot 240 and the second slot 243.

Referring back to FIGS. 1, 3 and 5, the control unit 8 is electrically coupled to the actuator 5 and the displacement detecting unit 6, is operable to output the control signal to the actuator 5, and is operable to identify the tissue type of the tissue layer based on the control signal and the displacement of the positioning reference 313 relative to the fixed reference (R) detected by the displacement detecting unit 6. To be more specific, the control unit 8 is operable to compare the detected displacement of the needle member 31 with a predetermined distance, so as to identify the tissue type of the tissue layer based on the comparison and the control signal. In this embodiment, the control unit 8 is, but not limited to, a microcontroller (MSP 430 2553G MCU) manufactured by Texas Instruments.

The monitoring unit 9 is communicatively coupled to the control unit 8.

The control unit 8 is operable to calculate a resistance force value of the tissue layer based on the control signal and the comparison and to transmit the resistance force value to the monitoring unit 9 for display.

Since compositions of the subcutaneous tissue layers of a biological body are different, density and hardness of the tissue layers are also different. For example, the density of muscle tissues is 1.12 and the density of fat tissues is 0.79 relative to the density of water. As such, different tissue layers exhibit different resistances to the needle tip 312 of the needle member 31 when being pierced. Therefore, as the needle member 31 penetrates into and passes through each of the tissue layers, changes in the resistance force values are significant and are noticeable to a human operator.

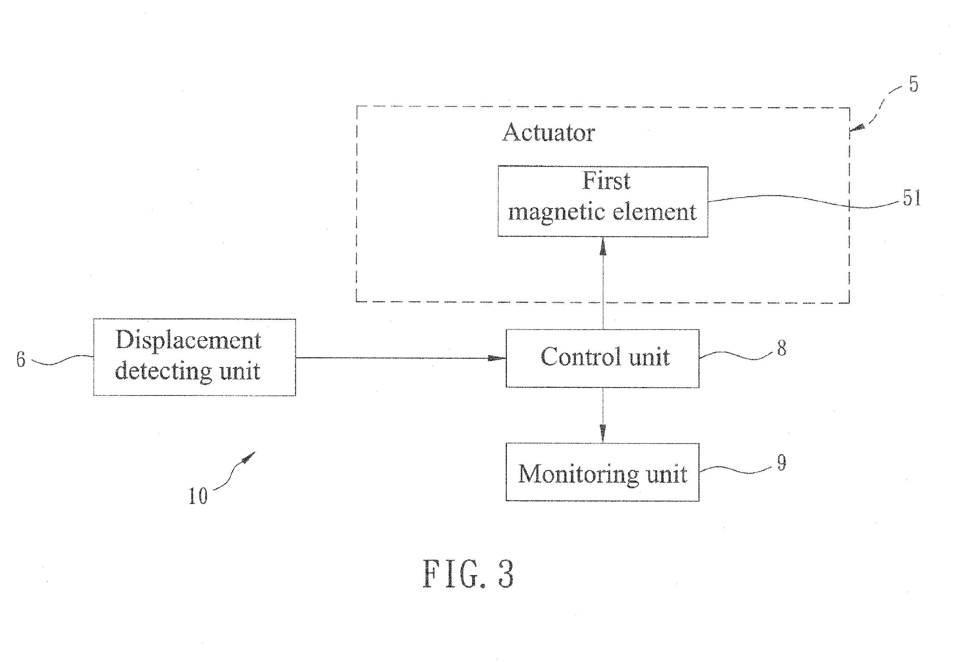

As shown in FIGS. 3, 5, 6 and 7, when in use, the needle tip 312 of the needle member 31 is initially aligned with the piercing end 321 of the outer needle guide 32, and the positioning reference 313 of the needle member 31 is aligned with the fixed reference (R) of the shell 2 (see FIG. 5). The control unit 8 is operable to output the control signal, which is in the form of a predetermined amplitude of an alternating current, to actuate the first magnetic element 51 of the actuator 5 to drive the needle member 31 to rapidly and repeatedly reciprocate between the protruding position and the retracting position. When the needle member 31 is driven by the actuator 5 to move the needle tip 312 toward the protruding position, the needle tip 312 protrudes out of the piercing end 321 of the outer needle guide 32 by a first protruding distance (L1) that corresponds to the displacement of the needle member 31 detected by the displacement detecting unit 6 (see FIG. 6). Subsequently, the needle member 31 is driven by the actuator 5 to move the needle tip 312 toward the retracting position, wherein the needle tip 312 is retracted back into the outer needle guide 32 by a retracting distance (L3) that corresponds to the displacement of the needle member 31 detected by the displacement detecting unit 6 (see FIG. 7).

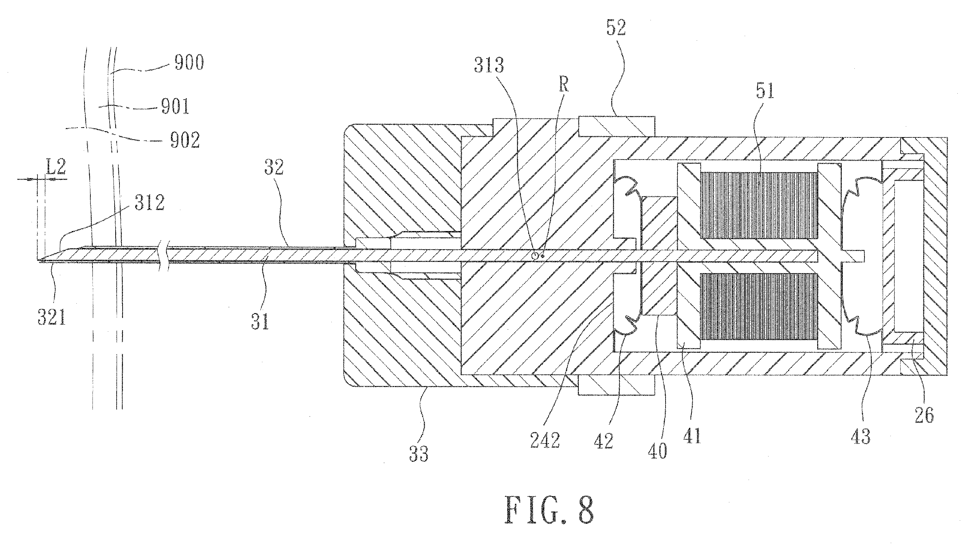

As shown in FIG. 8, when the outer needle guide 32 pierces through a skin layer 900, a fat layer 901, and reaches a muscle layer 902 of the biological body part, the needle tip 312 protrudes out of the piercing end 321 of the outer needle guide 32 by a second protruding distance (L2) that is smaller than the first protruding distance (L1) and that corresponds to the displacement of the needle member 31 detected by the displacement detecting unit 6(see FIG. 8). The control unit 8 then calculates the resistance force value of the muscle layer 902 based on the control signal and the comparison between the second protruding distance (L2) and the predetermined distance for identifying the tissue type, and then transmits the resistance force value calculated based on the comparison and the control signal to the monitoring unit 9 for display.

Since the needle member 31 is driven to repeatedly reciprocate between the protruding position and the retracting position, an average resistance force value can be calculated by the control unit 8, thereby increasing the accuracy of the identification of the tissue type.

As the needle tip 312 of the needle member 31 reaches the identified target tissue layer, the needle member 31 may be withdrawn, and a drug may be administered directly through the outer needle guide 32. In other applications, the needle member 31 may be withdrawn and replaced by a catheter for administration of a drug or a fluid into the target tissue layer, and the outer needle guide 32 may be later withdrawn over the catheter.

In this embodiment, the resistance force values are displayed directly on the monitoring unit 9. Alternatively, in other variations of this embodiment, the resistance force values may be displayed as letters, figures, etc. with no particular restriction based on a pre-established correlation between the resistance force values and the corresponding tissue types of the tissue layers. Alternatively, in other variations, the monitoring unit 9 may be a light-emitting member or a buzzer (not shown) which generates a notification when the resistance force value falls within a range of predetermined values corresponding to the target tissue layer.

In this embodiment, the first protruding distance (L1) and the retracting distance (L3) are equal and are set to be 1 mm, but may be adjusted based on different needs by adjusting the amplitude of the alternating current control signal. The waveform of the alternating current control signal may be, for example but not limited to, a sine wave, a square wave, a sawtooth wave, or a pulse wave.

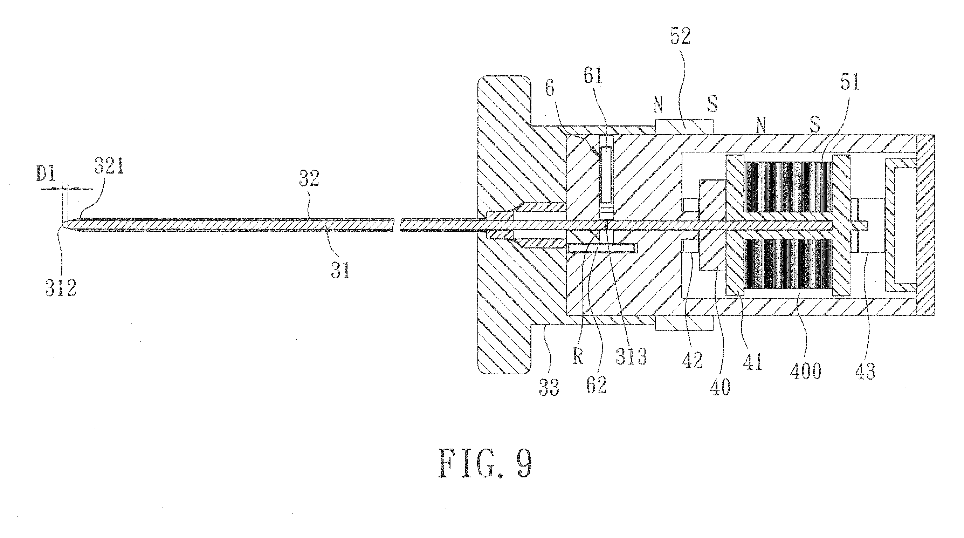

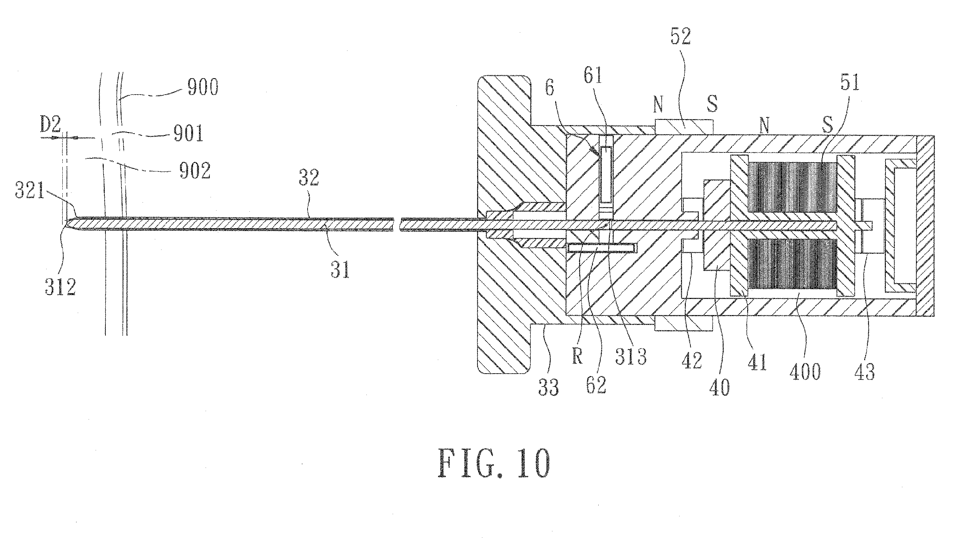

Referring to FIGS. 9 and 10, a second embodiment of the needle system according to the disclosure is shown to be similar to the first embodiment. The difference resides in that the control unit 8 is configured to adjust the control signal based on the detected displacement of the needle member 31, and to enable the actuator 5 to drive the needle member 31 to move the needle tip 312 back to the protruding position.

In this embodiment, the control unit 8 is operable to output the control signal in the form of a direct current. The first magnetic element 51 of the actuator 5 receives the control signal to drive the needle tip 312 of the needle member 31 to protrude out of the piercing end 321 of the outer needle guide 32 by a protruding distance (D1). At this time, the positioning reference 313 of the needle member 31 is aligned with the fixed reference (R) of the shell 2 and the needle member 31 is at the protruding position. When the outer needle guide 32 pierces through the skin layer 900 , the fat layer 901, and reaches the muscle layer 902 of the biological body, the needle tip 312 encounters different resistance forces from each of the tissue layers and is retracted back toward the outer needle guide 32 by different distances. As shown in FIG. 10, when the needle member 31 reaches the muscle layer 902, the needle tip 312 is retracted backward by a receding distance (D2) that is smaller than the protruding distance (D1) and that corresponds to the displacement of the needle member 31 detected by the displacement detecting unit 6. At this time, the control unit 8 increases the magnitude of the direct current to enable the actuator 5 to drive the needle member 31 to move the needle tip 312 back to the protruding position so as to minimize the displacement of the positioning reference 313 relative to the fixed reference (R). The control unit 8 is configured to calculate the resistance force value of the muscle layer 902 based on the adjusted control signal and the detected displacement and to transmit the calculated resistance force value to the monitoring unit 9 for display.

### [EXAMPLE]

Referring to FIGS. 3, 5 and 8, in an actual application for an epidural anesthetic procedure using the needle system of the first embodiment, the needle member 31 pierced through the skin, sequentially passed through the fat, the muscle, the periosteum membrane and the ligamentum flavum, and reached the epidural space (not shown). The average resistance force values of the aforementioned tissue layers were measured and monitored, and the results are shown in Table 1.

**Table 1**

| Tissue types | Average resistance force values |
|---|---|
| Skin | Not available |
| Fat | 36.74(gw)* |
| Muscle | 32.8(gw)** |
| Periosteum membrane | Not available |
| Ligamentum flavum | Not available |
| Epidural space | 0 (gw) |

| | |
|---|---|
| *Calculated based on five replicates: 35.9, 36.9, 37.0, 37.0 and 36.9. **Calculated based on five replicates: 34.0, 28. 9, 34.0, 32.9 and 34.0. | |

The average resistance force values for the skin and the periosteum membrane are not available because the skin and the periosteum membrane were too thin to be detected by the control unit 8 used in this example. As the needle member 31 breached the ligamentum flavum and entered the epidural space, the resistance value shown on the monitoring unit 9 decreased by a significant amount to 0 gw, thereby allowing the operator to confirm that the needle tip 312 of the needle member 31 has reached the epidural space for injection of anesthetics therein.

The needle system of the present disclosure can also be applied in, for example but not limited to, incisional biopsy using the outer needle guide 32 for sampling, and excisional biopsy using the outer needle guide 32 for removal of an entire lesion.

## Claims

1. A needle system capable of identifying tissue type of a tissue layer, the needle system being **characterized by**:
a needle device (1) for penetrating into a biological body part, the needle device (1) including
a needle member (31) that extends and is movable along a needle axis (X) and that has a needle tip (312), and
an electrically operable actuator (5) that is responsive to a control signal to drive the needle member (31) to move along the needle axis (X) against a resistance force from a tissue layer being penetrated by the needle device (1); and
a control module (10) including
a displacement detecting unit (6) that is operable to detect displacement of the needle member (31) along the needle axis (X), and
a control unit (8) that is electrically coupled to the actuator (5) and the displacement detecting unit (6), that is operable to output the control signal to the actuator (5), and that is operable to identify the tissue type of the tissue layer based on the control signal and the displacement of the needle member (31) detected by the displacement detecting unit (6).

2. The needle system according to Claim 1, **characterized in that** the needle device (1) further includes an outer needle guide (32) that extends along the needle axis (X) to surround the needle member (31) and that has a piercing end (321) configured to penetrate into the biological body part, the needle member (31) being movable relative to the outer needle guide (32) between a protruding position, where the needle tip (312) of the needle member (31) protrudes out of the piercing end (321) of the outer needle guide (32), and a retracting position, where the needle tip (312) of the needle member (31) is retracted back into the outer needle guide (32).

3. The needle system according to Claim 2, further **characterized in that**, when the needle member (31) is driven by the actuator (5) to move the needle tip (312) toward the protruding position, the needle tip (312) protrudes out of the piercing end (321) of the outer needle guide (32) by a protruding distance that corresponds to the displacement of the needle member (31) detected by the displacement detecting unit (6), the control unit (8) being operable to compare the detected displacement of the needle member (31) with a predetermined distance, so as to identify the tissue type of the tissue layer based on the comparison and the control signal.

4. The needle system according to Claim 3, further **characterized by** a monitoring unit (9) that is communicatively coupled to the control unit (8), the control unit (8) being operable to calculate a resistance force value of the tissue layer based on the control signal and the comparison and to transmit the resistance force value to the monitoring unit (9) for display.

5. The needle system according to Claim 3, further **characterized in that** the control unit (8) is operable to adjust the control signal based on the displacement of the needle member (31), and to enable the actuator (5) to drive the needle member (31) to move the needle tip (312) back to the protruding position.

6. The needle system according to Claim 2, further **characterized in that** the actuator (5) drives the needle member (31) to reciprocate between the protruding position and the retracting position.

7. The needle system according to Claim 2, further **characterized in that** the needle device (1) further includes a shell (2) that is fixedly mounted to the outer needle guide (32) oppositely of the piercing end (321) and that receives the actuator (5) therein.

8. The needle system according to Claim 7, further **characterized in that**:
the shell (2) has a fixed reference (R);
the needle member (31) further has a receiving portion (311) that is disposed in the shell (2) and that is provided with a positioning reference (313); and
the displacement detecting unit (6) detects the displacement of the needle member (31) based on displacement of the positioning reference (313) relative to the fixed reference (R).

9. The needle system according to Claim 8, further **characterized in that** the displacement detecting unit (6) includes a light emitting member (61) disposed adjacent to the positioning reference (313), and a light detecting member (62) disposed corresponding in position to the light emitting member (61) and the positioning reference (313) for detecting displacement of the positioning reference (313) relative to the fixed reference (R).

10. The needle system according to Claim 9, further **characterized in that** the positioning reference (313) is configured as a radially-extending hole, the shell (2) further including a front wall (24) through which the needle member (31) extends, the front wall (24) having
a surrounding surface (245) that surrounds the needle axis (X) and that is formed with a first slot (240) which extends radially inward and which accommodates the light emitting member (61) therein, and
a front end surface (241) that is formed with a second slot (243) which is opposite to the first slot (240) in a direction transverse to the needle axis (X), which extends away from the outer needle guide (32) and which accommodates the light detecting member (62), and a connecting slot (244) which is formed between the positioning reference (313) of the needle member (31) and the second slot (243), the first slot (240) spatially communicating with the second slot (243) via the positioning reference (313) and the connecting slot (244).

11. The needle system according to Claim 7, further **characterized in that** the actuator (5) includes a first magnetic element (51) that is connected to the needle member (31), and a second magnetic element (52) that is mounted to the shell (2) and that cooperates with the first magnetic element (51) for driving movement of the needle member (31), one of the first magnetic element (51) and the second magnetic element (52) being configured as a coil for receiving the control signal from the control unit (8).

12. The needle system according to Claim 11, further **characterized in that** the needle device (1) further includes a biasing unit (4) that is connected to the needle member (31) and that biases the needle member (31) against a driving force provided by the actuator (5).

13. The needle system according to Claim 12, further **characterized in that** the biasing unit (4) is disposed in the shell (2) and includes a connecting member (41) that is connected to the needle member (31) and that is slidable along the needle axis (X) relative to the shell (2), and first and second resilient members (42, 43) that are respectively disposed on two opposite sides of the connecting member (41) along the needle axis (X), the first resilient member (42) having two opposite ends respectively and persistently abutting against the connecting member (41) and the shell (2), the second resilient member (43) having two opposite ends respectively and persistently abutting against the connecting member (41) and the shell (2).

14. The needle system according to Claim 13, further **characterized in that** the connecting member (41) includes a cylinder body (411) that extends along the needle axis (X) and that surrounds the needle member (31), and two side plates (412) that are respectively connected to two opposite ends of the cylinder body (411) and that cooperate with the cylinder body (411) to confine an accommodating space (400) for accommodating the first magnetic element (51).

15. A needle device **characterized by**:
a shell (2) having front and rear ends (21,22) opposite to each other along a needle axis (X);
a needle unit (3) including
an outer needle guide (32) that is mounted to the front end (21) of the shell (2), that extends along the needle axis (X), that spatially communicates with the shell (2) and that has a piercing end (321) configured to penetrate into a biological body part, and
a needle member (31) that is disposed in the shell (2) and the outer needle guide (32), that has a needle tip (312) protruding from the front end (21) of the shell (2) and that is movable relative to the shell (2) and the outer needle guide (32); and
an electrically operable actuator (5) that is responsive to a control signal to drive the needle member (31) and move the needle tip (312) between a protruding position, where the needle tip (312) protrudes out of the piercing end (321) of the outer needle guide (32), and a retracting position, where the needle tip (312) is retracted back into the outer needle guide (32).
